# EUROPEAN PATENT SPECIFICATION

(11) **EP 3 095 724 B1**
(45) Date of publication and mention of the grant of the patent: **04.07.2018**
(21) Application number: 16169341.1
(22) Date of filing: 12.05.2016
(51) Int. Cl.: B65D 75/52, B65D 25/54, B65D 27/04, A61M 25/00, B65D 75/36, A61B 17/06, A61M 25/01

(54) **A CATHETER ASSEMBLY AND A METHOD FOR PREPARING A PACKAGE OF A CATHETER ASSEMBLY**
KATHETERANORDNUNG UND VERFAHREN ZUR HERSTELLUNG EINER VERPACKUNG EINER KATHETERANORDNUNG
ENSEMBLE DE CATHÉTER ET PROCÉDÉ DE PRÉPARATION D'UN EMBALLAGE D'UN ENSEMBLE DE CATHÉTER

(30) Priority: 22.05.2015 DK 201570305
(43) Date of publication of application: 23.11.2016
(73) Proprietor: MBH-International A/S, 3450 Allerød (DK)
(72) Inventor: Schønfeldt, Lars, 3000 Helsingør (DK)
(74) Representative: Holme Patent A/S

(56) References cited:
- EP-A2- 1 160 174
- WO-A1-96/30277
- US-A- 3 930 580
- US-A- 4 106 621
- US-A- 5 803 260
- US-A1- 2007 051 652
- US-A1- 2009 045 093

## Description

The present invention relates to a catheter assembly comprising a catheter and a package with a compartment for accommodating the catheter, as well as a method for preparing a package of a catheter assembly.

The catheter can be any type of catheter, such as a urinary catheter used for emptying the bladder, diagnosing pathology in the lower urinary tract, and monitoring urine output.

For all types of catheters it is important to avoid bacteria contamination of the catheter before and during its insertion into the body. An important goal is thus to be able to manufacture a catheter assembly with a sterile environment inside the catheter package.

The main problem caused by urinary catheters is infections in the urethra, bladder, or the kidneys - known as urinary tract infections, which usually need to be treated with antibiotics. Other problems are also well known. Infections from urinary catheters are one of the major sources of the total number of infections hitting patients staying in hospitals.

The present invention deals with reducing the risk of contaminating a catheter when opening the catheter package, removing the catheter from the package and inserting it into the body.

A urinary catheter, and other types of catheters, has a catheter tip, a catheter shaft and a grip end. For urinary catheters the catheter tip is either inserted through the urethra or through a small opening made in the lower tummy.

In order to reduce the risk of contamination it is very important that the person performing the steps of opening the package, removing the catheter there from and subsequently inserting it into the body does not touch the catheter tip or the catheter shaft, but only touches the intended part (grip end) of the catheter.

It is generally known in the art that a catheter package in which a catheter is located can be provided with a text or a mark at an appropriate location, so that the person opening the package can identify the orientation of the catheter inside the package even before the package is opened.

However, such a person - whether it is a doctor, nurse or the actual person into which the catheter is to be inserted - can fail to notice or just fail to perceive such a text/mark on the package, and thus possibly handle the opening of the package and/or the removal of the catheter from the package in an incorrect manner, which could contaminate the catheter.

The type of catheter inside the catheter assembly according to the present invention may be a ready-to-use urinary catheter, where said catheter comprises on at least a part of its surface an exterior hydrophilic coating arranged to reduce the coefficient of friction after treatment with a liquid swelling medium. It is preferred that the catheter is a urinary catheter e.g. an intermittent or indwelling catheter with an exterior hydrophilic coating or surface layer, and which is stored in the activated state. For such types of catheter relevant information regarding the state of the catheter is e.g. that the hydrophilic coating is properly wetted, i.e. activated so that the catheter is ready to be used.

Relevant information regarding the state of a urinary catheter is e.g. whether is it broken, the catheter size (e.g. indicated by the Connector color of the catheter), and the water content/level inside the compartment (no leakage indicates sterility and proper activation of the hydrophilic coating).

Hence there is a need for a catheter assembly that provides the user with an indication of the orientation and/or state of the catheter inside the closed package that can be perceived by the human brain in an easier and more intuitive manner than with the known indications, such as the above-mentioned text/mark on the package.

The novel and unique way whereby this is achieved according to the present invention is defined by claim 1. The at least one transparent part provides the person opening the package with a simple and very intuitive way of identifying the orientation of the catheter before the package is opened, as it is the actual catheter, and the optionally liquid swelling medium, that is at least partly visible though the transparent part of the packaging and not just a text/marking on a totally non-transparent catheter package. Being able to actually see the catheter itself provides the advantage that orientation of the catheter is easier perceived by the person opening the package due to the way the human brain perceives objects. To interpret information regarding the orientation of such a catheter is much easier for the human brain if the catheter is visible, as the brain otherwise would have to convert the visual perception of a text/marking on a package into the actual orientation of the catheter inside the package. The at least one transparent part provides also a simple and intuitive way of identifying the state of the catheter before the package is opened, as the human perception of such information is much easier than if the catheter is behind a package of a non-transparent material. If the catheter is behind a non-transparent package the only way can try to identify the state of the catheter, before the package is opened, is by using the sense of touch.

Known catheter packages does not have any transparent parts, which makes it impossible to see the interior of an unopened package, and they are normally made of one or more non-transparent materials.

The package of the catheter assembly of the present invention is made of at least one non-transparent material and at least one transparent material, where at least one part of the package does not comprise a non-transparent material and thus is transparent.

The at least one non-transparent part of the package of the present invention can be provided with text and drawings in order to provide the person using the catheter with miscellaneous information regarding e.g. the content of the package, instructions for use of the catheter assembly, and/or the orientation of the catheter inside the package. According to the present invention, an otherwise non-transparent package is provided with at least one transparent window formed by a corresponding number of openings in the non-transparent material of the package, where each of the one or more openings are sealingly covered by one or more of the at least one transparent materials of the package.

A simple version of such a catheter package with one transparent window can be formed by two sheets of a non-transparent material, where the two sheets are connected to each other by a joint along their periphery to define a package with a compartment for accommodating a catheter, where one of the two sheets is provided with an opening that is sealingly covered by a third sheet of a transparent material. The opening preferably has an appropriate shape, appropriate dimensions and has an appropriate location on the package for a person to be able to visually identify the orientation and/or the state of the catheter inside such a closed package.

In the simple version with one transparent window, the window can have a substantially rectangular shape extending about 35 mm in the longitudinal direction of the package and about 10 mm in the transverse direction of the package, in which case the package can have a longitudinal length of about 500 mm and a width of about 38 mm in the transverse direction, however other shapes and dimensions of the transparent window are also contemplated within the scope of the present invention.

In more advanced versions, one or both of the two sheets can be formed by two or more non-transparent materials or by a mix of non-transparent and transparent materials, and the third sheet can be formed by one or more transparent materials.

Each of the openings can be sealingly covered by a sheet of transparent material by providing such a sheet with a shape and dimensions that not only covers the opening by also partly extends over the non-transparent part of the package to an appropriate degree.

The term "sheet" as used herein is to be understood as either a sheet or a film, which both can be multi-layered.

Instead of, or in addition to, providing the package with one or more transparent windows, the at least one transparent part may comprise a transparent part extending from one end of the package towards the opposite end of the package, where the extent is at least 25% of the end-to-end length of the package. Such a transparent part can provide visual access to the entire area in the vicinity of e.g. the grip end of the catheter, which especially is advantageous as catheters inside a catheter package normally is able to move around to some extend.

In another alternative embodiment the at least one transparent part comprises a transparent part extending along the entire length of the catheter package or at least along the length of the compartment for accommodating the catheter, where said transparent part has a transverse extent (e.g. 10 mm) adapted to allow a person to be able to visually identify the orientation and/or the state of the catheter inside such a closed package.

It is preferred that the transparent material extends over at least a portion of the non-transparent material along the entire border(s) between the transparent part(s) and the non-transparent part(s) of the package, so that the transparent material and the non-transparent material can be sealingly connected to each other along said entire border(s).

If the package is provided with a transparent window as described above, a weld can be provided in the overlapping area between the transparent material and the non-transparent material along the entire periphery of the opening. If the package is provided with a transparent part extending over at least 25 % of the end-to-end length as described above, a weld can be provided in the overlapping area along the entire border between the transparent part and the non-transparent part.

In a preferred embodiment of the present invention an adhesive, such as a pressure sensitive adhesive, is located between the transparent material and the non-transparent material in the overlapping portion(s). The adhesive is advantageously located at an appropriate distance from the border(s) between the transparent part(s) and the non-transparent part(s) to ensure that leachables of adhesive material does not enter the compartment comprising the catheter and the optionally liquid swelling medium.

The adhesive preferably forms a continuous seal along but at an appropriate distance from the border(s) between the transparent part(s) and the non-transparent part(s), where said continuous seal ensures that the compartment in which the catheter is located is sealed off to keep the catheter sterile.

In the embodiment with the adhesive, the overlapping portion(s) of the transparent material that is/are not adhered to the non-transparent part(s) by means of the adhesive is/are preferably welded to the non-transparent part(s), so that the entire overlapping portion(s) of the transparent material is securely attached to the non-transparent part(s) of the package. This has the advantage that the materials of the package are less likely to become unintentionally detached from each other.

The adhesive has the function of preventing that the transparent material dislocates away from the intended position in relation to the opening in the non-transparent material before or during the process of attaching (e.g. by means of welding) the transparent material to the non-transparent material at the overlapping portion(s) there between.

In the embodiment where the adhesive forms a continuous seal at an appropriate distance from the border(s) between the transparent part(s) and the non-transparent part(s) it is preferred that the welded area comprises a first welded portion between the inner edge of the continuous seal and the opening, and a second welded portion between the outer edge of the continuous seal and the edge of the transparent material.

By welding around the area(s) with adhesive material, such as along both edges of the above-mentioned continuous seal, is obtained the advantage that the adhesive material is boxed in so that no leachables of adhesive material can enter the compartment comprising the catheter and the optionally liquid swelling medium.

It is contemplated within the scope of the present invention that the adhesive could be replaced with any means (e.g. a means that provides sufficient friction between the transparent material and the non-transparent material) that prevents the transparent material from dislocating away from the intended position in relation to the opening in the non-transparent material before or during the process of attaching (e.g. by means of welding) the transparent material to the non-transparent material at the overlapping portion(s) there between.

The transparent material and/or the non-transparent material are preferably a sheet or a film.

The transparent material is preferably one or more medical grade thermoplastic film materials, such as polyethylene and/or polypropylene, which preferably has a thickness of 80 - 100 my.

As an example, a catheter assembly with a transparent window as described above, where the transparent material that covers the opening is a PE-film with a thickness of 80 - 100 my, and where the window has a substantially rectangular shape with a length of 35 mm and a width of 10 mm, would have a moisture vapor transmission rate (MVTR) of about 3,5 g/m²/24hr and a water loss/evaporation of around 10% w/w within 36 months.

It is preferred that the shape and dimensions of the at least one transparent window are adapted to the shape/dimensions of the catheter and the shape/dimensions of the compartment accommodating the catheter, so that visual identification of the catheter tip, the grip end, or both is possible independent of the position of the catheter inside the package.

It is preferred that the at least one transparent material is gas permeable and the at least one non-transparent material is gas impermeable, at ambient temperature and atmospheric pressure, with respect to gasses such as water vapor, O₂, CO₂, N₂ and ethylene oxide. The gas permeability of the transparent material is preferred in order to provide a simple and inexpensive embodiment, even though this will allow a limited amount of any liquid swelling medium to evaporate from the closed package during storage. The amount of swelling medium that will evaporate from the packaging will however not affect the chemical environment and thus the state of the catheter inside the package, as the amount of liquid swelling medium inside the packaging easily can be adjusted accordingly. This is especially important when a catheter with a hydrophilic surface/coating is packed in the wetted, i.e. activated condition, since the arrangement of the packaging according to the invention will ensure that the hydrophilic catheter will remain in the activated stage during the entire shelf life of said packaging, i.e. said catheter will remain activated for periods between a few months and up to five years.

It will be understood that that all materials of the packaging are arranged for preventing any liquid, in the liquid stage, to either enter or exit said packaging.

The catheter package can be formed by two sheets or films, as mentioned earlier in this application. Each of the sheets/films is preferably either a single sheet/film or a laminate consisting of multiple layers of sheets/films, made from one or more thermoplastic materials or composites. One of the layers in the laminate can advantageously be a metallic layer, e.g. an aluminium foil, providing the liquid-impermeability, gas-impermeability, and non-transparentability. However, other means for obtaining a similar effect is known for a person skilled in the art, and are also contemplated within the scope of the present invention. A plastic layer, preferably of a thermoplastic polymer, can advantageously be provided as the outermost layer facing away from the package, as such a plastic layer can be provided with text and illustrations to be seen by the person handling the catheter assembly.

The present invention also relates to a method of preparing a package for a catheter assembly according to the present invention as defined by the appended claims, where the method comprises the following steps:
- providing a first sheet and a second sheet made of a non-transparent material,
- making at least one opening in the first sheet and/or the second sheet,
- attaching at least one sheet of transparent material to the first sheet and/or the second sheet in a sealingly manner so that the at least one opening is covered by the at least one sheet of transparent material, and
- joining the first sheet and the second sheet together to form a package with a compartment for accommodating a catheter.

By making the opening (s) in one or both sheets before the sheets are joined to form a package is obtained the advantage that the sheet (s) into which an opening is to be made can be located on a flat surface and in a flat state when the opening is made, which production wise is highly preferably. The same advantage apply to the step of attaching the at least one sheet of transparent material.

For each sheet of transparent material that is attached to the first sheet or second sheet according to the method of the present invention, an adhesive (or any means that provides sufficient friction between the transparent material and the non-transparent material) can be provided between the transparent material and the first sheet or second sheet as described above.

The first sheet and the second sheet are preferably joined by means of welding to obtain a welded joint, where the welded joint may comprise a part provided as a peelable joint permitting at least partly separation of the sheets from each other for withdrawal of the catheter from the package. However, other techniques for joining the first sheet and the second sheet are also contemplated within the scope of the preset invention.

The package of the catheter assembly can be made from two sheets of non-transparent material that are joined together as described above, but is also contemplated within the scope of the present invention that the package can be made from a single sheet of non-transparent material that is folded to form two halves, which can be positioned on top of each other and joined together to form a package with a compartment for accommodating a catheter.

The invention will be explained in greater detail below, describing only exemplary embodiments of the catheter assembly as shown in the figures 1 - 4, where
Fig. 1 shows a top view of a catheter assembly where the package is provided with one transparent window,
Fig. 2 shows a bottom view of the catheter assembly in fig. 1,
Fig. 3 shows a side view of the catheter assembly in figs. 1 and 2,
Fig. 4 is a fractional view of the catheter assembly in fig. 1 showing the transparent window, and
Fig. 5 illustrates a step in the method of preparing a package for a catheter assembly, where the package is provided with a transparent window.

The catheter package of the catheter assemblies according to the figures are shown and described as being elongated with one transparent window at one end of the compartment, which accommodates the catheter (not shown). However other shapes and dimensions of the catheter package as well as other shapes, dimensions and locations of the at least one transparent part on the package are within the scope of the present invention.

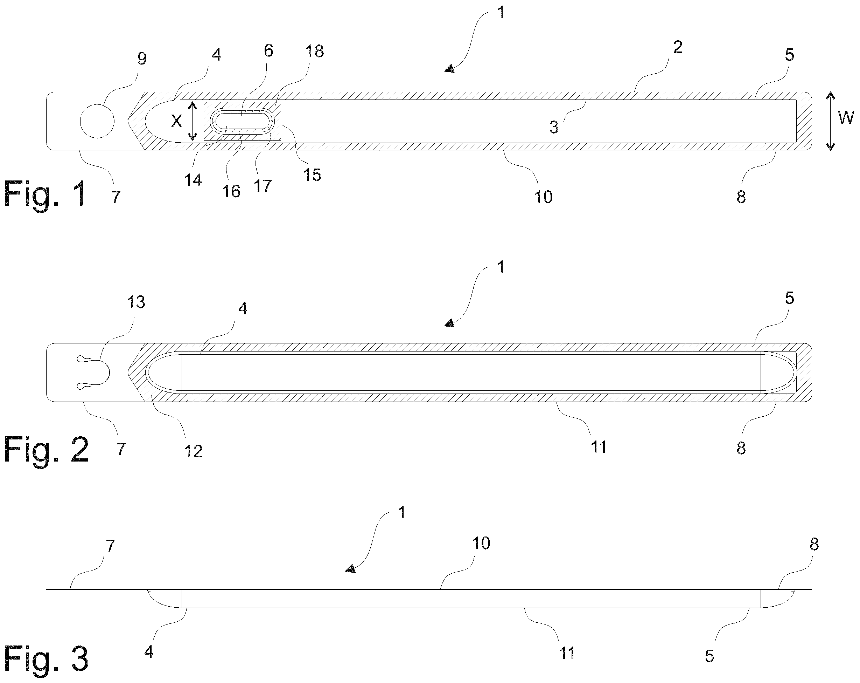

Fig. 1 shows a catheter assembly 1 consisting of an elongated catheter package 2 with an elongated compartment 3 in which a catheter (not shown) is accommodated.

The compartment 3 has a first end 4 and a second end 5, where a transparent window 6 is located close to the first end 4. The catheter is intended to be orientated with its grip end at the first end 4 of the compartment 3 and its tip at the second end 5 of the compartment 3.

The package 2 has a first end 7 and a second end 8, where a sticky dot 9 is located at the first end 7 to enable a user to hang the catheter assembly from an appropriate surface at the location where the catheter is to be used, such as next to a toilet. The sticky dot 9 is arranged for being able to stick to most common surfaces.

As shown in figs. 1 - 3 the package 2 consists basically of an upper sheet 10 of non-transparent material and a lower sheet 11 of non-transparent material that are joined together to form a package 2 with a compartment 3 for accommodating a catheter. The upper sheet 10 is provided with the transparent window 6 as shown in fig. 1. These two sheets 10, 11 of non-transparent material are joined together at the hatched area 12 as shown in figs. 1 and 2 and thereby form the elongated compartment 3.

The part of the package 2 at the first end 7 where the two sheets 10, 11 of transparent material are not joined together (i.e. the not-hatched part in figs. 1 - 2) enables a user to grab hold of one of the two sheets 10, 11 with one hand and the other of the two sheets 10, 11 with the other hand in order to pull the two sheets 10, 11 apart at the first end 4 of the compartment 3 in an easy and conveniently manner.

As shown in fig. 2 the second sheet 11 is provided with a horseshoe grip 13 that enables a user to pull the two sheets of non-transparent material apart at the first end 4 of the compartment 3 is an easy and conveniently manner, as the user can put one finger through the horseshoe grip to obtain a firm grip of the second sheet 11 during the pulling.

The transparent window 6, the sticky dot 9 and the horseshoe grip 13 are conveniently located at the first end 7 of the package 2 where the grip end of the catheter is located, which also is the end of the package 2 that the user is supposed to open when removing the catheter.

The sticky dot 9 as well as the horseshoe grip 13 are optional features of the present invention.

The upper sheet 10 of non-transparent material is provided with an opening 14 as shown in fig. 1. The transparent window 6 is formed by attaching a rectangular sheet 15 of transparent material to the non-transparent material of the package 2 so that the rectangular sheet 15 of transparent material not only covers the opening 14 by also partly extends over the non-transparent material of the package 2. Other shapes and dimensions of the opening 14 and/or the sheet 15 of transparent material are within the concept of the present invention.

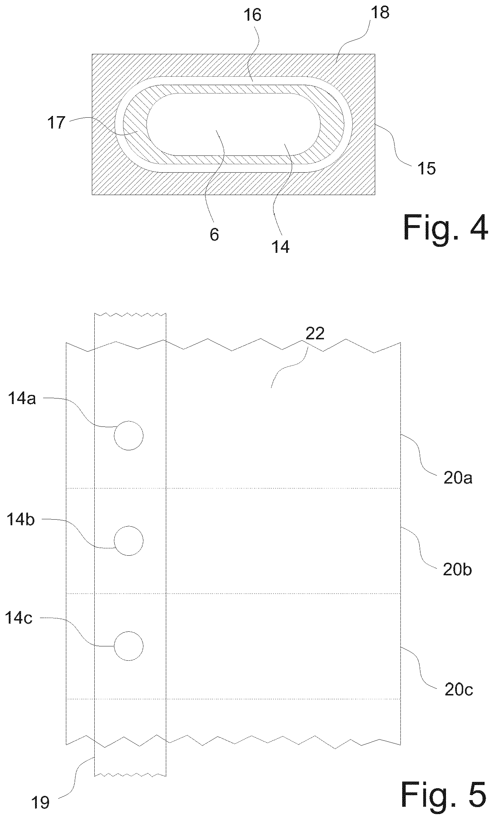

As most clearly shown in fig. 4 the rectangular sheet 15 of transparent material is attached to the non-transparent material of the package 2 by means of an adhesive 16, such as a pressure sensitive adhesive, provided there between at a appropriate distance from the border of the opening 14 and forming a continuous seal along said border, and by welding the remaining overlapping portion of the rectangular sheet 15 to the non-transparent material of the package 2. A first welded portion 17 is located between the periphery of the opening 14 and the inner edge of the adhesive 16. A second welded portion 18 is located between the periphery of the rectangular sheet 15 of transparent material and the outer edge of the adhesive 16.

As mentioned in connection with figs. 1 - 3, the package 2 consists basically of two sheets 10, 11 of non-transparent material that are joined together to form the package 2 with the compartment 3. These sheets 10, 11 can advantageously be manufactured from a roll of bulk film of the chosen non-transparent material from which the individual sheets 10, 11 are cut out.

Fig. 5 illustrates a production step in the method of preparing a catheter package for a catheter assembly with a transparent window according to the present invention, similar to the catheter assembly 1 shown in figs. 1 - 3.

There is a slight difference between the catheter assembly 1 shown in figs. 1 - 4 and the catheter assembly to be manufactured from a method including the production step illustrated in fig. 5, where said difference is width of the sheet 15 of transparent material in relation to the width W of the package 2.

The width X of the sheet 15 of transparent material of the embodiment shown in figs. 1 - 4 is less than the width W of the package 2. In the embodiment shown in fig. 5 the transparent material extents over the entire width W of the catheter package, as will be described below.

Fig. 5 shows a part of a bulk film 19 ready to be cut along the cut lines 21a, 21b, 21c, 21d, so that a number of individual sheets 20a, 20b, 20c each having the width W are formed. Prior to the cutting step, the openings 14a, 14b, 14c are formed in the bulk film 19 by any appropriate technique such as punching, and a bulk film 22 of transparent material is positioned on top of the bulk film 19 of non-transparent material so that the openings 14a, 14b, 14c are covered by the bulk film 22 of transparent material as shown in fig. 5.

Also prior to the cutting step, the bulk film 22 of transparent material is attached to the bulk film 19 of non-transparent material using e.g. the adhesive and welding technique as described in connection with figs. 1 - 4.

Once the two bulk films 19, 22 are attached to each other, the individual sheets 20a, 20b, 20c are cut out by cutting along the cutting lines 21a, 21b, 21c, 21d.

The manufacturing of a catheter package using the production step illustrated in fig. 5, where it is a bulk film 22 of a transparent material and a bulk film 19 of a non-transparent material that are attached to each other before the individual sheets 20a, 20b, 20c are cut out, is faster and thus more economic than if the individual sheets were cut out before the were attached to each other.

## Claims

1. Catheter assembly (1) comprising:
- a catheter comprising on at least a part of its surface an exterior hydrophilic coating,
- an elongated package (2) with an elongated compartment (3) for accommodating the catheter,
**characterized in that**
- the compartment (3) comprises a liquid swelling medium,
- the package (2) comprises at least one non-transparent part made of at least one non-transparent material, and at least one transparent part made of at least one transparent material, said transparent part is arranged for allowing a visual identification of the orientation and/or state of the catheter in the package (2),
- the package (2) comprises at least one transparent window (6) formed by a corresponding number of openings (14, 14a, 14b, 14c) in the non-transparent material where the at least one opening (14, 14a, 14b, 14c) is sealingly covered by the transparent material, and
- one of the at least one transparent windows (6) is located at one end of the compartment (3).

2. Catheter assembly (1) according to claim 1, **characterized in that** the transparent material extends over at least a portion of the non-transparent material along the entire at least one border between the at least one transparent part and the at least one non-transparent part.

3. Catheter assembly (1) according to claim 2, **characterized in that** an adhesive (16), such as a pressure sensitive adhesive, is located between the transparent material and the non-transparent material in the at least one overlapping portion along the at least one border between the at least one transparent part and the at least one non-transparent part.

4. Catheter assembly (1) according to claim 3, **characterized in that** the at least one overlapping portion of the transparent material that is not adhered to the at least one non-transparent part is welded to the at least one non-transparent part.

5. Catheter assembly (1) according to claim 3 or 4, **characterized in that** the adhesive (16) forms a continuous seal along but at an appropriate distance from the at least one border between the at least one transparent part and the at least one non-transparent part.

6. Catheter assembly (1) according to claim 5, **characterized in that** the welded area comprises a first welded portion (17) between the inner edge of the continuous seal (16) and the opening (14, 14a, 14b, 14c), and a second welded portion (18) between the outer edge of the continuous seal (16) and the edge of the transparent material.

7. Catheter assembly (1) according to any of claims 1 - 6, **characterized in that** the transparent material is a transparent film and/or sheet.

8. Catheter assembly (1) according to any of claims 1 - 7, **characterized in that** the transparent material is medical grade thermoplastic film material, such as polyethylene or polypropylene.

9. Catheter assembly (1) according to any of claims 1 - 8, **characterized in that** the shape and dimensions of the at least one transparent window (6) are adapted so that visual identification of the catheter tip, the grip end or both is possible independent of the position of the catheter inside the package (2).

10. Catheter assembly (1) according to any of claims 1 - 9, **characterized in that** the catheter is a ready-to-use urinary catheter.

11. Method of preparing a package (2) of the catheter assembly (1) according to any of the claims 1 - 10, comprising:
- providing a first sheet (10) and a second sheet (11) made of a non-transparent material,
- making at least one opening (6) in the first sheet (10) and/or the second sheet (11),
- attaching at least one sheet (15) of transparent material to the first sheet (10) and/or the second sheet (11) in a sealingly manner so that the at least one opening (6) is covered by the at least one sheet (15) of transparent material to form a transparent window (14), and
- joining the first sheet (10) and the second sheet (11) together to form a package (2) with a compartment (3) for accommodating a catheter.

12. Method according to claim 11, **characterized in that** the step of attaching a sheet (15) of transparent material to the first sheet (10) or the second sheet (11) comprises an adhesive (16), such as a pressure sensitive adhesive, provided between the transparent material (15) and the first sheet (10) or second sheet (11) at an appropriate distance from the periphery of the at least one opening (6), where the adhesive (16) prevents the transparent material (15) from dislocating away from the intended position in relation to the at least one opening (6) in the non-transparent material.

13. Method according to claim 11 or 12, **characterized in that** the sheet (10, 22) with the at least one transparent window (6) is prepared using the steps of:
- providing a bulk film (19) of non-transparent material,
- making appropriate openings (14a, 14b, 14c) in said bulk film (19),
- positioning a bulk film (22) of transparent material on top of the bulk film (19) of non-transparent material so that the openings (14a, 14b, 14c) are covered by the bulk film (22) of transparent material,
- attaching the two bulk films (19, 22) to each other, and
- cutting out individual sheets (20a, 20b, 20c) with at least one transparent window (6) by cutting along appropriate cutting lines (21a, 21b, 21c, 21d).

## Patentansprüche

1. Katheteranordnung (1), umfassend:
- einen Katheter, umfassend, auf mindestens einem Teil seiner Oberfläche, eine äußere hydrophile Beschichtung,
- eine längliche Packung (2) mit einem länglichen Fach (3) zur Unterbringung des Katheters,
**dadurch gekennzeichnet, dass**
- das Fach (3) ein flüssiges Quellmedium umfasst,
- die Packung (2) mindestens einen nicht transparenten Teil aus mindestens einem nicht transparenten Material und mindestens einen transparenten Teil aus mindestens einem transparenten Material umfasst, wobei der transparente Teil angeordnet ist, um eine visuelle Identifizierung der Ausrichtung und/oder des Zustands des Katheters in der Packung (2) zu ermöglichen,
- die Packung (2) mindestens ein transparentes Fenster (6) umfasst, das durch eine entsprechende Anzahl von Öffnungen (14, 14a 14b, 14c) in dem nicht transparenten Material gebildet wird, wobei die mindestens eine Öffnung (14, 14a, 14b, 14c) abdichtend von dem transparenten Material bedeckt ist, und
- eines des mindestens einen transparenten Fensters (6) an einem Ende des Fachs (3) angeordnet ist.

2. Katheteranordnung (1) nach Anspruch 1, **dadurch gekennzeichnet, dass** sich das transparente Material über mindestens einen Abschnitt des nicht transparenten Materials entlang der gesamten mindestens einen Grenze zwischen dem mindestens einen transparenten Teil und dem mindestens einen nicht-transparenten Teil erstreckt.

3. Katheteranordnung (1) nach Anspruch 2, **dadurch gekennzeichnet, dass** ein Klebstoff (16), wie ein druckempfindlicher Klebstoff, zwischen dem transparenten Material und dem nicht transparenten Material in dem mindestens einen sich überschneidenden Abschnitt entlang der mindestens einen Grenze zwischen dem mindestens einen transparenten Teil und dem mindestens einen nicht transparenten Teil angeordnet ist.

4. Katheteranordnung (1) nach Anspruch 3, **dadurch gekennzeichnet, dass** der mindestens eine sich überschneidende Abschnitt des transparenten Materials, der nicht an dem mindestens einen nicht transparenten Teil klebt, mit dem mindestens einen nicht transparenten Teil verschweißt ist.

5. Katheteranordnung (1) nach einem der Ansprüche 3 oder 4, **dadurch gekennzeichnet, dass** der Klebstoff (16) eine kontinuierliche Abdichtung entlang, aber in einem angemessenen Abstand von der mindestens einen Grenze zwischen dem mindestens einen transparenten Teil und dem mindestens einen nicht-transparenten Teil bildet.

6. Katheteranordnung (1) nach Anspruch 5, **dadurch gekennzeichnet, dass** der geschweißte Bereich einen ersten geschweißten Abschnitt (17) zwischen dem inneren Rand der kontinuierlichen Abdichtung (16) und der Öffnung (14, 14a, 14b, 14c) und einen zweiten geschweißten Abschnitt (18) zwischen dem äußeren Rand der kontinuierlichen Abdichtung (16) und dem Rand des transparenten Materials umfasst.

7. Katheteranordnung (1) nach einem der Ansprüche 1 bis 6, **dadurch gekennzeichnet, dass** das transparente Material ein(e) transparente(r) Film und/oder Folie ist.

8. Katheteranordnung (1) nach einem der Ansprüche 1 bis 7, **dadurch gekennzeichnet, dass** das transparente Material ein thermoplastisches Filmmaterial medizinischer Güteklasse ist, wie Polyethylen oder Polypropylen.

9. Katheteranordnung (1) nach einem der Ansprüche 1 bis 8, **dadurch gekennzeichnet, dass** die Form und die Abmessungen des mindestens einen transparenten Fensters (6) so ausgelegt sind, dass eine visuelle Identifizierung der Katheterspitze, des Griffendes oder beider unabhängig von der Position des Katheters in der Packung (2) möglich ist.

10. Katheteranordnung (1) nach einem der Ansprüche 1 bis 9, **dadurch gekennzeichnet, dass** der Katheter ein gebrauchsfertiger Blasenkatheter ist.

11. Verfahren zur Herstellung einer Packung (2) der Katheteranordnung (1) nach einem der Ansprüche 1 bis 10, umfassend:
- Bereitstellen einer ersten Folie (10) und einer zweiten Folie (11) aus einem nicht transparenten Material,
- Herstellen mindestens einer Öffnung (6) in der ersten Folie (10) und/oder der zweiten Folie (11),
- Befestigen mindestens einer Folie (15) aus transparentem Material an der ersten Folie (10) und/oder der zweiten Folie (11) in abdichtender Weise, sodass die mindestens eine Öffnung (6) von der mindestens einen Folie (15) aus transparentem Material bedeckt ist, um ein transparentes Fenster (14) zu bilden, und
- Zusammenfügen der ersten Folie (10) und der zweiten Folie (11) zu einer Packung (2) mit einem Fach (3) zur Unterbringung eines Katheters.

12. Verfahren nach Anspruch 11, **dadurch gekennzeichnet, dass** der Schritt des Befestigens einer Folie (15) aus transparentem Material an der ersten Folie (10) oder der zweiten Folie (11) einen Klebstoff (16), wie einen druckempfindlichen Klebstoff, umfasst, der zwischen dem transparenten Material (15) und der ersten Folie (10) oder der zweiten Folie (11) in einem angemessenen Abstand vom Umfang der mindestens einen Öffnung (6) bereitgestellt ist, wobei der Klebstoff (16) das transparente Material (15) daran hindert, sich von der beabsichtigten Position in Bezug auf die mindestens eine Öffnung (6) in dem nicht-transparenten Material zu verschieben.

13. Verfahren nach Anspruch 11 oder 12, **dadurch gekennzeichnet, dass** die Folie (10, 22) mit dem mindestens einen transparenten Fenster (6) unter Verwendung der folgenden Schritte hergestellt wird:
- Bereitstellen eines Dünnfilms (19) aus nicht transparentem Material,
- Herstellen geeigneter Öffnungen (14a, 14b, 14c) in dem Dünnfilm (19),
- Positionieren des Dünnfilms (22) aus transparentem Material auf dem Dünnfilm (19) aus nicht transparentem Material, sodass die Öffnungen (14a, 14b, 14c) von dem Dünnfilm (22) aus transparentem Material bedeckt sind,
- Zusammenfügen der zwei Dünnfilme (19, 22) und Ausschneiden einzelner Folien (20a, 20b, 20c) mit
- mindestens einem transparenten Fenster (6) durch Schneiden entlang angemessener Schnittlinien (21a, 21b, 21c, 21d).

## Revendications

1. Ensemble cathéter (1) comprenant :
- un cathéter comprenant, sur au moins une partie de sa surface, un revêtement hydrophile externe ;
- un conditionnement allongé (2) comprenant un compartiment allongé (3) pour loger le cathéter ;
**caractérisé en ce que**
- le compartiment (3) comprend un milieu liquide gonflant ;
- le conditionnement (2) comprend au moins une partie non transparente constituée d'au moins une matière non transparente, et au moins une partie transparente constituée d'au moins une matière transparente, ladite partie transparente étant conçue pour permettre une identification à l'oeil nu de l'orientation et/ou de l'état du cathéter dans le conditionnement (2) ;
- le conditionnement (2) comprend au moins une fenêtre transparente (6) formée par un nombre correspondant d'ouvertures (14, 14a, 14b, 14c) dans la matière non transparente, ladite au moins une ouverture (14, 14a, 14b, 14c) étant recouverte en étanchéité par la matière transparente ; et
- une desdites au moins une fenêtre transparente (6) est disposée à une extrémité du compartiment (3).

2. Ensemble cathéter (1) selon la revendication 1, **caractérisé en ce que** la matière transparente s'étend par-dessus au moins une portion de la matière non transparente le long de la totalité d'au moins une limite entre ladite au moins une partie transparente et ladite au moins une partie non transparente.

3. Ensemble cathéter (1) selon la revendication 2, **caractérisé en ce qu'**un adhésif (16), tel qu'un adhésif sensible à la pression, est disposé entre la matière transparente et la matière non transparente dans ladite au moins une portion chevauchante le long de ladite au moins une limite entre ladite au moins une partie transparente et ladite au moins une partie non transparente.

4. Ensemble cathéter (1) selon la revendication 3, **caractérisé en ce que** la au moins une portion chevauchante de ladite matière transparente qui n'adhère pas à la au moins une partie non transparente est soudée à ladite au moins une partie non transparente.

5. Ensemble cathéter (1) selon la revendication 3 ou 4, **caractérisé en ce que** l'adhésif (16) forme un joint d'étanchéité continu le long de ladite au moins une limite entre ladite au moins une partie transparente et ladite au moins une partie non transparente, mais à une distance appropriée de ladite limite.

6. Ensemble cathéter (1) selon la revendication 5, **caractérisé en ce que** la zone soudée comprend une première portion soudée (17) entre le bord interne du joint d'étanchéité continu (16) et l'ouverture (14, 14a, 14b, 14c) et une seconde portion soudée (18) entre le bord externe du joint d'étanchéité continu (16) et le bord de la matière transparente.

7. Ensemble cathéter (1) selon l'une quelconque des revendications 1 à 6, **caractérisé en ce que** la matière transparente est un film transparent et/ou une feuille transparente.

8. Ensemble cathéter (1) selon l'une quelconque des revendications 1 à 7, **caractérisé en ce que** la matière transparente est une matière pelliculaire thermoplastique de qualité médicale, telle que du polyéthylène ou du polypropylène.

9. Ensemble cathéter (1) selon l'une quelconque des revendications 1 à 8, **caractérisé en ce que** la forme et les dimensions de ladite au moins une fenêtre transparente (6) sont conçues pour que l'identification à l'oeil nu de la pointe du cathéter, de l'extrémité de prévention ou des deux soit possible indépendamment de la position du cathéter à l'intérieur du conditionnement (2).

10. Ensemble cathéter (1) selon l'une quelconque des revendications 1 à 9, **caractérisé en ce que** le cathéter est un cathéter urinaire prêt à l'emploi.

11. Procédé de préparation d'un conditionnement (2) de l'ensemble cathéter (1) selon l'une quelconque des revendications 1 à 10, comprenant le fait de :
- procurer une première feuille (10) et une seconde feuille (11) constituées d'une matière non transparente ;
- pratiquer au moins une ouverture (6) dans la première feuille (10) et/ou dans la seconde feuille (11) ;
- fixer au moins une feuille (15) de matière transparente à la première feuille (10) et/ou à la seconde feuille (11) en étanchéité d'une manière telle que ladite au moins une ouverture (6) est recouverte par ladite au moins une feuille (15) de matière transparente pour former une fenêtre transparente (14); et
- joindre la première feuille (10) et la seconde feuille (11) l'une à l'autre pour former un conditionnement (2) comprenant un compartiment (3) pour loger un cathéter.

12. Procédé selon la revendication (11), **caractérisé en ce que** l'étape de fixation d'une feuille (15) de matière transparente à la première feuille (10) ou à la seconde feuille (11) comprend un adhésif (16), tel qu'un adhésif sensible à la pression, que l'on prévoit entre la matière transparente (15) et la première feuille (10) ou la seconde feuille (11) à une distance appropriée par rapport à la périphérie de ladite au moins une ouverture (6), dans lequel l'adhésif (16) empêche la matière transparente (15) de se déloger par rapport à la position recherchée par rapport à ladite au moins une ouverture (6) dans la matière non transparente.

13. Procédé selon la revendication 11 ou 12, **caractérisé en ce que** la feuille (10, 22) comprenant ladite au moins une fenêtre transparente (6) est préparée en utilisant les étapes consistant à :
- procurer une bande de film (19) constituée d'une matière non transparente ;
- pratiquer des ouvertures appropriées (14a, 14b, 14c) dans ladite bande de film (19) ;
- positionner une bande de film (22) constituée d'une matière transparente par-dessus la bande de film (19) constituée d'une matière non transparente d'une manière telle que les ouvertures (14a, 14b, 14c) sont recouvertes par la bande de film (22) constituée d'une matière transparente ;
- fixer l'une à l'autre les deux bandes de films (19, 22) ; et
- découper des feuilles individuelles (20a, 20b, 20c) comprenant au moins une fenêtre transparente (6) en procédant à une découpe le long de lignes de coupe appropriées (21a, 21b, 21c, 21d).
